# EUROPEAN PATENT SPECIFICATION

(11) **EP 0 801 577 B1**
(45) Date of publication and mention of the grant of the patent: **13.03.2002**
(21) Application number: 94930444.8
(22) Date of filing: 19.09.1994
(51) Int. Cl.: A61M 5/00, A61M 16/00, A61M 16/04

(54) **APPARATUS FOR VENTILATING AND ASPIRATING**
GERÄT ZUM VENTILIEREN UND ASPIRIEREN
APPAREIL POUR VENTILATION ET D'ASPIRATION

(43) Date of publication of application: 22.10.1997
(73) Proprietor: Sorenson Medical, Inc., Salt Lake City UT 84115 (US)
(72) Inventor: COLES, Peter, W., H., Salt Lake City, UT 84105 (US); BLOCK, Randall, D., Salt Lake City, UT 84108 (US); SCHUMANN, Paul, A., Centerville, UT 84014 (US); SORENSON, James, Levoy, Salt Lake City, UT 84115 (US)
(74) Representative: Smulders, Theodorus A.H.J., Ir.
(86) International application number: US9410505
(87) International publication number: WO9609082

(56) References cited:
- EP-A- 0 466 334
- US-A- 5 029 580
- US-A- 5 309 902
- US-A- 5 325 851
- US-A- 5 333 606
- PATENT ABSTRACTS OF JAPAN vol. 016, no. 265 (C-0951), 16 June 1992 & JP 04 064348 A (MATSUSHITA ELECTRIC IND CO LTD), 28 February 1992,

## Description

### BACKGROUND OF THE INVENTION

Field: This invention relates to means for ventilating and aspirating the respiratory tracts of medical patients. It is particularly directed to an improved ventilation circuit adapter.

State of the Art: Currently available means for ventilating and aspirating congested lungs and obstructed breathing passage ways may be classified as either "simple" or "complex." Devices of both classes have significant drawbacks and limitations. The available devices of simple construction tend to expose both the patient and the service provider to microbial contaminants. Although these devices are relatively less expensive, their use potentially provides dangerously inadequate patient ventilation during the aspirating procedure. More elaborate devices tend to be physically obstructive and economically prohibitive to use.

In common medical practice, a medical practitioner inserts a tracheal suctioning catheter into a medical patient's lungs. The catheter may be positioned within a tracheal tube, endotracheal tube or nasopharyngeal tube intubated at a tracheotomy incision, mouth or nasal opening, respectively. The end of the catheter accessible by the medical practitioner is regarded as its "proximal" end. The end of the catheter remote from the practitioner, that is, within the patient, is regarded as its "distal" end. In a typical procedure, negative pressure is exerted at the proximal end of the catheter. Undesired respiratory fluids and mucoid secretions are thereby evacuated from the medical patient through the distal end of the catheter.

In a relatively simple practice, a source of vacuum pressure remains attached to the proximal catheter end. Negative pressure is not communicated to the distal catheter end initially. Rather, negative pressure is communicated to an aperture near the proximal catheter end. When aspiration of the patient is desired, the practitioner plugs the aperture, normally with a finger or thumb. Negative pressure is thereby instantaneously diverted from the small aperture near the proximal catheter end through the catheter lumen to the distal end of the catheter. In this fashion, undesired respiratory fluids are aspirated from the respiratory tract of the patient. The suctioning catheter is then disconnected from the vacuum source and discarded after a single use.

Ventilation of the patient simultaneously with this aspirating procedure is generally impractical. Such ventilation inherently requires assistance from additional personnel as well as mechanical assistance, neither of which is available as a matter of course. Accordingly, the aspirating procedure must ordinarily be completed within a short period of time. Otherwise, the patient may experience oxygen deficiency and be exposed to the resulting potential for heart arrhythmia and/or failure.

More complex aspirating devices are available, which while avoiding some of the disadvantages attendant to the structurally more simple devices, impose additional disadvantages. For example, they are significantly more expensive to construct, and their greater number of moving parts increases the risk of disfunction during use. They are also larger and more cumbersome to use, imposing a greater physical interference with other, related procedures.

The complex, closed-system devices are characteristically comprised of a manifold structure enabling introduction of ventilating gases and intermittent exhalation of patient breath simultaneously with insertion and operation of the tracheal suctioning catheter. These systems almost universally involve at least one collapsible, plastic envelope entirely surrounding the catheter and purporting to provide a sterile barrier between the otherwise exposed external surface of the catheter and the ambient atmosphere. In practice, a practitioner manually externally collapses the envelope onto the external surface of the catheter and advances the catheter into the throat of a patient, retracting the catheter in a similar fashion following the aspiration procedure.

These closed-system devices under present medical protocol are ordinarily used at least hourly for up to 48 hours for each patient before being replaced. Problems attendant to such frequent and repeated in-dwelling use are numerous, among other problems constriction of the catheter lumen and valve flow path with dried and drying phlegm and other respiratory secretions, creation of contaminant-allowing pinholes in the collapsible plastic barrier and the need to physically move the lengthy and cumbersome devices from one location on the body of the patient to another when the devices interfere with other procedures. Associated with the dried and drying secretions inside the lumen are like secretions on the exterior of the catheter wall which accumulate at the manifold wiper seal; such thick and undesired respiratory secretions not only restrict the facile movement of the catheter through the manifold, but also can be unavoidably reintroduced to the patient in subsequent repeat procedures.

Material prior art structures and methods are described among other places in United States Patent Numbers 5,133,345 to Lambert; 5,107,829 to Lambert; 5,083,561 to Russo; 5,065,754 to Jensen; 5,029,580 to Radford et al.; 5,025,806 to Palmer et al.; 4,981,466 to Lambert; 4,967,743 to Lambert; 4,938,741 to Lambert; 4,872,579 to Palmer; 4,838,255 to Lambert; 4,836,199 to Palmer; 4,834,726 to Lambert; 4,825,859 to Lambert; 4,805,611 to Hodgkins; 4,696,296 to Palmer; 4,638,539 to Palmer; 4,569,344 to Palmer; 4,327,723 to Frankhouser; and 3,991,762 to Radford. US-A-5,333,606 discloses the features of the preamble of claim 1 of the present invention.

Other closed-system, sterility preserving devices which involve sterile barriers formed of rubber-like materials and intended for penetration by rigid cannulae include: rubber like caps fitted on "y" injection site structures and located at the end of indwelling intravenous catheters, such as those distributed by HARMAC Medical Specialties, Inc. of Buffalo, New York under the product number H1429104; rubber-like caps fitted on housing structure anchorable at injection sites, such as those distributed by Baxter Healthcare Corp. of Deerfield, Illinois under product number 2N3399 and their trademark "InterLink(TM);" and threaded lock cannula used in conjunction with the Baxter InterLink(TM) for needleless injection by Becton Dickinson & Co. of Franklin Lakes, New Jersey under product number 303369 and their trademark "InterLink(TM)."

United States Letters Patent No. 4,351,328 to Bodai discloses a method and apparatus for accomplishing endotracheal suctioning of a patient without the need for disconnecting the patient from a respirator. The disclosed apparatus permits the maintenance of positive end expiratory pressure without interruptions during suctioning. A notable feature of the device is the ease with which a suctioning tube may be removed from an associated ventilation manifold.

There remains a need for a ventilating and aspirating device, wherein a closed-system, multiple use manifold for a single patient is coupled with a single-patient, single use catheter, which structure is comprised of relatively few parts, is easily assembled and is not susceptible to disfunction.

Also, a need remains for a tracheal suctioning device which unobtrusively enables ongoing closed-system ventilation and repeated aspiration of a single patient without interim obstruction of peripheral procedures.

### SUMMARY OF THE INVENTION

The present invention provides an improved apparatus for simultaneously ventilating and aspirating a medical patient. It may be embodied as a sealed ventilation circuit adapter that enables simultaneous patient ventilation and safe tracheal suctioning.

The adapter of this invention may be embodied to accommodate any of a wide variety of commercially available suction catheters. Catheters with flared tips, obvious depth markings, and normally closed vacuum control valves are presently considered to be most useful. Currently preferred embodiments of the adapter comprise two primary components: a manifold assembly, and a catheter carrier. The manifold assembly will ordinarily be attached to a patient for an extended period, typically 24-48 hours. The catheter carrier may be constructed as a single procedural use disposable device; it is ideally constructed to accept interchangeably a variety of commercially available catheters.

The invention enables a practitioner to insert a flexible catheter tube into either lung of a patient in one smooth motion without risk of contamination or infection and without assistance from others. In some embodiments, a practitioner is able to activate vacuum pressure with either hand and without assistance from others.

A standard flexible tracheal suctioning catheter tube is attached at the proximal (practitioner) end to a fitting cylinder for insertion into a vacuum source. The cylinder includes an offset opening which when plugged transfers communication of the vacuum pressure from outside the catheter to the internal lumen of the catheter. The distal (patient) end of the catheter tube is structured to permit respiratory secretions to be suctioned into the lumen of the catheter tube.

The catheter tube is slidable lengthwise through a passageway in a multifunction manifold positioned at the distal end of the apparatus. The manifold preferably includes a port at the distal end for attachment to and communication with an indwelling intubation device, such as a tracheal tube, endotracheal tube or nasopharyngeal tube. The manifold also may include a ventilating structure extending radially from and in fluid communication with the passageway, for selectively introducing ambient air, oxygenated air and other therapeutic gasses into the respiratory system of the patient. Other conduits may also be provided for the introduction of other suitable gases and lavage solutions to the respiratory system.

Specifically, this invention provides an advantageous ventilation circuit adapter for interfacing a suction catheter with an intubation device. The adapter may be visualized as comprising two major components; specifically, a manifold assembly and a catheter carrier. In practice, the adaptor may be associated in a package or kit with one or more catheters. Alternatively, a manifold assembly may be associated with a plurality of catheter carriers.

The manifold generally has a body with an open interior. A distal end portion of the body is adapted to couple with an intubation device. A proximal end portion of the body is formed as an open port defined by a continuous wall. The manifold may take various forms, but its proximal end portion should be approximately axially aligned with its distal end portion so that a catheter may be inserted through the proximal end portion to exit from the distal end portion. A directional barrier is carried by the proximal end portion of the manifold assembly. It is constructed and arranged to effect a seal against fluid flow through the open interior of the manifold towards the proximal end portion but to pass a catheter introduced through the proximal end portion.

Preferably, the directional barrier is configured to be inserted in the proximal end portion in sealing relationship with the open interior, and comprises a normally closed valving structure. The normally closed valve structure may be constructed and arranged to effect a sliding seal arrangement with the external surface of a catheter passed through the body of the manifold; that is, from the proximal end portion towards the distal end portion through the valve structure. Preferably, the valve and catheter carrier are mutually adapted to effect this sliding seal. The valve structure may carry detection means constructed and arranged to signal the precise location of the tip of a catheter positioned within the valve structure. For example, these means may signal, by resistance to travel or sound, the presence of the tip when it is moved to the proximity of the valve structure from a location closer to the distal end portion of the manifold assembly.

The manifold assembly desirably includes a ventilation port in fluid flow communication with the open interior of the manifold body. The central axis of the ventilation port will ordinarily be oriented transverse the central axes of the proximal end portion and the distal end portion of the manifold. A lavage port may also be provided in fluid flow communication with the open interior. The central axis of the lavage port should also be oriented transverse the central axes of the proximal end portion and the distal end portion.

The catheter carrier typically includes a leading end portion adapted to couple with the proximal end portion of the manifold and a trailing end portion carrying a catheter introducer structure. The introducer structure is constructed and arranged to interface with the directional barrier when the leading end portion of the carrier is coupled with the proximal end portion of the manifold. For example, it may comprise an introducer tip constructed as a continuous wall to define an interior passageway configured to receive and pass a catheter and an exterior surface configured to interface with the directional barrier. The directional barrier is further preferably structured to effect a resilient seal against an internal surface of the continuous wall. The interior passageway may include an entry constructed and arranged to effect a sliding seal with the exterior surface of a catheter. In some embodiments, the exterior surface of the introducer tip and the directional barrier are mutually adapted to effect a sealed relationship when the leading end portion of the catheter carrier is coupled with the proximal end portion of the manifold body.

The directional barrier may be structured to effect a resilient seal both against an internal surface of the open interior of the manifold body and between the proximal end portion of the body and the catheter carrier.

A sputum collector may be carried by the trailing end portion of the catheter carrier. This collector functions to accumulate sputum fluids and the like wiped from a catheter as it is withdrawn through the barrier seal. In one embodiment, the collector includes a chamber positioned in approximate axial alignment with the distal end portion opposite the manifold with respect to the leading end portion. A proximal closing member for the chamber carries an entry approximately axially aligned with the introducer tip. The closing member may be structured to effect a sliding seal with the exterior surface of a catheter.

The chamber is desirably structured as a hollow cylindrical extension from the trailing end portion of the carrier. The closing member is desirably structured as an end panel of the extension with a central opening adapted to receive a catheter in friction-slipping engagement. The term "friction slipping," as used in this disclosure, refers to a slidable association in which the exterior surface of the catheter is engaged about its circumference by the interior surface of the central opening of the end panel. Fluids adhering to the catheter are thus removed by a wiping action as the catheter is withdrawn through the opening. The end panel conveniently comprises a removable cap structured and arranged to effect a sealed closure of the cylindrical extension.

In use, the manifold assembly is interposed between a patient's indwelling tube at the distal end of the manifold and a ventilating circuit. These junctions preferably embody a swivel configuration to permit left or right bedside placement of the ventilation circuitry, and free rotation of the ventilation circuit with patient head movement to reduce the risk of extubation.

The directional barrier may be configured as an elongate pressure and sterility barrier, and the normally closed valve may be in the shape of a duckbill, with a normally closed central aperture interposed within the passageway of the manifold. Whether the barrier is structured to include an aperture of elongate transverse dimension or a simple slit in a membranous end of the barrier wall, the barrier in combination with the adapter assembly provides an effective seal against patient expectoration, contaminant migration and pressure leakage.

The catheter carrier may be configured as an assembly surrounding the catheter, including an introducer tip and a concentric outer introducer housing connected at one end by an adapter base. The introducer housing fits snugly over the outside of the proximal end of the manifold. As the introducer housing is slid over the manifold, first coupling structure carried by the external surface of the manifold engages second coupling structure associated with the internal wall of the introducer housing. The first and second coupling structures are mutually adapted to allow the practitioner to slide the carrier assembly and manifold toward one another until the manifold is seated against the adapter base. Then the housing is rotated with respect to the carrier to positively lock the adapter and manifold together during use of the device.

The introducer housing may be slid over the proximal end of the manifold. An initial sterile barrier is thereby created. After the initial barrier is created, the introducer housing is further slid toward the manifold, causing the introducer tip to penetrate the aperture of the directional barrier. Though this penetration may preliminarily partially compromise the aperture seal, the initial sterile barrier between the manifold and carrier maintains a closed-system environment within the manifold. After the introducer tip has penetrated the directional barrier, the catheter can be advanced, with minimal frictional resistance, through the manifold and indwelling tube into either lung of the patient.

Once the catheter and adapter are attached to the manifold, a presuction saline lavage is typically introduced through the lavage port in the manifold which directs the respiratory secretion flow into the patient tube through the distal end of the manifold. The lavage port is purposefully located immediately adjacent to the patient tube and with a non-perpendicular fluid injection angle so as to optimally direct the saline flow into the patient tube with little risk of back flow into the ventilation circuit. One preferred manifold design incorporates a window through which a practitioner may view the catheter, its suctioned contents and any depth markings which may be printed on the catheter.

While the practitioner is performing the evacuation procedure, the catheter may be repeatedly inserted and retracted with selective introduction of lavage fluid through a lavage port on the manifold. The manifold is configured to prevent lavage fluid from draining through the ventilation port, obviating the need for the manifold to be tilted during lavage and thereby averting the potential for disconnection of the manifold from the indwelling endotracheal tube.

Upon repeated advancement and retraction of the catheter during an evacuation procedure, undesired respiratory secretions are prevented from flowing into the ventilation port or back toward the patient. Such secretions are squeegeed off of the external surface of the catheter during retraction.

The squeegee function is accomplished by a wiper seal snugly surrounding the catheter within the proximal end of the adapter assembly. The wiper seal has two primary functions: to clean the external catheter wall during retraction so as to minimize practitioner exposure to patient contaminants; and to maintain a pressure seal around the catheter, ensuring a continuous closed, leak tight ventilation circuit. The preferred wiper seal configuration interfaces with the catheter tube with low frictional resistance, facilitating facile catheter insertion.

A sputum collector compartment is located between the wiper seal and the base of the adapter to accumulate the undesired secretions. The sputum collector or trap has two primary functions. It traps the wiper seal between the wiper trap and the adapter housing, and acts as a catheter guide during insertion and retraction of the catheter.

Upon completion of an evacuation procedure, a practitioner retracts the catheter. In some arrangements, a structure, such a flare at the distal tip of the catheter, catches against a reducing constrictor in the catheter passageway within the introducer tip. This flare to reducing constrictor contact further enhances and maintains the closed-system environment within the manifold by providing a positive stop which prevents a practitioner from inadvertently retracting a catheter from the manifold and catheter carrier.

After the practitioner has performed the respiratory evacuation procedure, the introducer housing may be axially rotated in the direction opposite to the direction it was turned at the beginning of the procedure, thereby to decouple the carrier assembly so that it can be retracted from the manifold.

As the carrier assembly is being retracted from the manifold, the introducer housing maintains a closed-system seal, even as the introducer tip within is pulled through the directional barrier. After the aperture in the directional barrier is closed, thereby maintaining a pressure and sterility barrier, the introducer housing is retracted proximally off from the manifold. The single use catheter and adapter assembly may then be dropped onto the sterile field barrier atop the patient, together with the surgical gloves, and the sterile field may be wrapped around the gloves, catheter and adapter assembly and discarded appropriately, leaving the closed-system, single-patient manifold in place for later multiple uses.

A dust cap, typically tethered to the manifold, may be placed over the proximal end of the manifold between suction procedures. The dust cap acts as a protective sterile barrier between the suction procedures as well as a seal against pressure differentials across the manifold to prevent back flow through the normally closed directional barrier.

It is significant that throughout the entirety of a respiratory evacuation procedures, a sterile, closed-system environment is maintained within the manifold. This invention thus makes it possible for such respiratory evacuation procedures to be accomplished without the need for ancillary personnel assistance. An individual respiratory nurse or technician can perform the entire procedure.

Further, it is significant that this invention provides means whereby throughout the entirety of such procedures, ongoing oxygenation or other ventilation of the patient can be maintained, without the need for repeated disconnection, connection or other moving of ventilating tubes and other auxiliary equipment.

It is also significant that the manifold, comprising the more costly portion of the adapter of this invention, unobtrusively remains attached to the patient and is reusable for numerous additional suctioning procedures with single-use catheter and carrier assemblies of relatively negligible expense.

### BRIEF DESCRIPTION OF THE ILLUSTRATED DRAWINGS

In the drawings, which illustrate what is currently regarded as the best mode for carrying out the invention:
FIG. 1 is a partially exploded and partially broken away cross-sectional view in elevation of a preferred embodiment of the invention in disassembled condition;
FIG. 2 is an enlargement of a portion of FIG. 1;
FIG. 3 is a partial sectional view of the manifold assembly;
FIG. 4 is a view similar to FIG. 1, but showing the components in assembled condition;

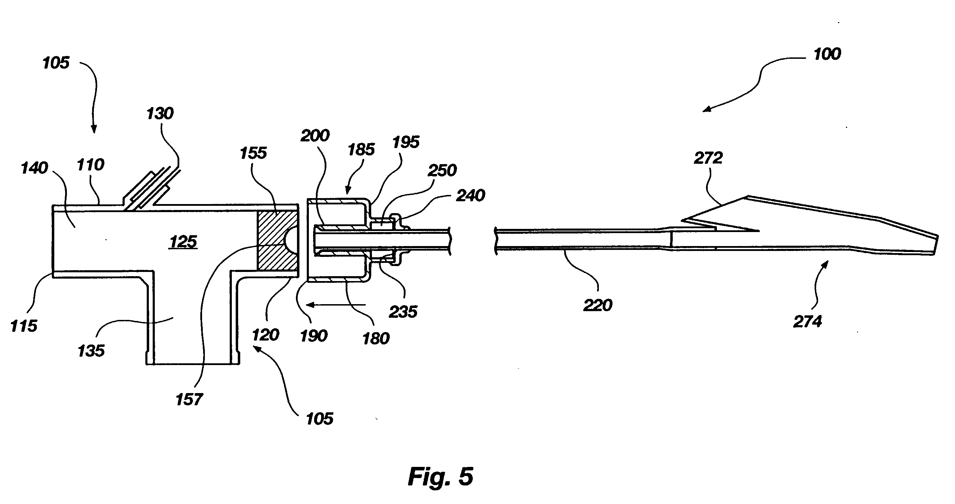

FIG. 5 is a partially exploded and partially broken away cross-sectional view similar to FIG. 1 of an alternative embodiment of the invention; and
FIG. 6 is a view similar to FIG. 5, showing the components in assembled condition.

### DETAILED DESCRIPTION OF THE PREFERRED EMBODIMENT

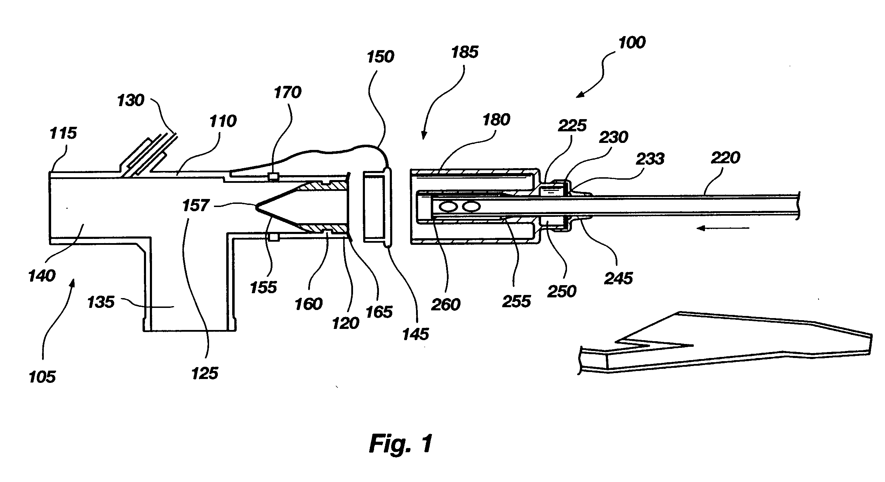

Figures 1-4, illustrate one configuration of a device, designated generally 100, embodying the invention. A manifold assembly, designated generally 105, comprises a body 110 with a distal end 115 and a proximal end 120. The body 110 defines a space 125 between the distal end 115 and proximal end 120. A lavage port 130 located on the body 110 of the manifold assembly 105 opens into and is in communication with the manifold space 125. Similarly, a ventilation port 135 located on the body 110 is in communication with the manifold space 125. Located at the distal end 115 of the body 110 is an endotracheal port 140 opening toward the patient and attachable to an indwelling fixture (not shown), such as a tracheal, endotracheal or nasopharyngeal tube.

A cap 145, capable of covering the proximal end 120 of the body 110, may be attached to the body 110 with a tether 150. Situated within the space 125 of the body 110 is a directional barrier 155 preferably formed of a pliant, durable, rubber-like material with strong dimensional memory. The directional barrier 155 includes a central aperture 157. The directional barrier 155 is seated around its entire cross-sectional periphery against a rim 160 extending inwardly from the internal wall of the body 110. The barrier 155 and the rim 160 interface in a manner that achieves a sealing and anchoring of the directional barrier 155 relative to the body 110. A seal extension 165 may protrude slightly from the directional barrier 155 axially beyond the proximal end 120 of the body 110 to provide additional sealing effect between the cap 145 and the body 110 when the cap 145 is in position to cover the proximal end 120 of the body 110.

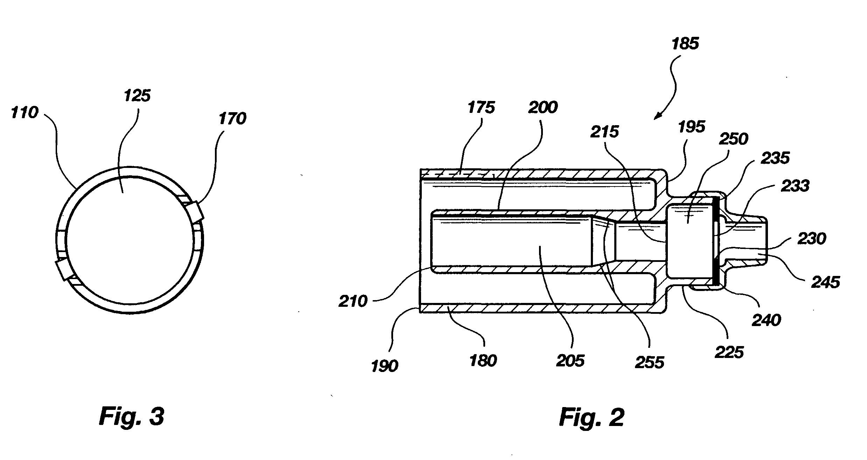

At least one positioning and locking post 170 extends radially outward from the body 110 of the manifold assembly 105. The post 170 interacts with a locking channel 175 formed in the internal wall of the introducer housing 180 of a catheter carrier 185, as illustrated more clearly in Figure 2. The channel 175 extends axially from the carrier front 190 in the direction of the carrier base 195, providing relative positioning of the manifold assembly 105 and the catheter carrier 185. The channel 175 then continues circumferentially at approximately a right angle from the axially directed channel 175, providing a means of locking the manifold assembly 105 and the catheter carrier 185 together when the post 170 has been moved axially and then radially within the channel 175. The seal extension 165 is shown configured to seal against the internal wall of the introducer housing 180 as the catheter carrier 185 and manifold assembly 105 are in the process of being assembled. The seal extension 165 is further structured to seal against the carrier base 195 when the catheter carrier 185 is locked to manifold assembly 105. In this attached position, the resilience of the seal extension 165 biases the carrier 185 axially to maintain locking pressure between the locking post 170 of the manifold assembly 105 and the channel 175 of the introducer housing 180.

The catheter carrier 185 comprises an introducer tip 200 situated concentric with and inside of the introducer housing 180. The introducer tip 200 is seated against, and may be formed integrally with the carrier base 195. A catheter passageway 205 is formed through the length of the introducer tip 200, opening at the leading edge 210 and through a catheter opening 215 in the carrier base 195. The catheter opening 215 may be formed with a cross-sectional dimension slightly larger than but corresponding to the outside cross-sectional dimension of a tracheal suctioning catheter 220.

Trap walls 225 extend axially from, and are formed integrally with, the carrier base 195. A pliant and resilient washer-like wiper seal 230 with a hole 233 therethrough is placed against the distal edge 235 of the trap walls 225. A trap cap 240 is attached to the trap walls 225 and holds the wiper seal 230 in place. The trap cap 240 includes a support opening 245 which provides catheter guidance and relief of strain on the catheter 220 imposed by non-axial forces encountered during insertion and retraction of the catheter 220. In combination, the wiper seal 230, the trap cap 240 and the trap wall 225 contribute to the sterility and pressure barrier function of the manifold 185 during use. The trap walls 225, together with the wiper seal 230 and the trap cap 240, define a sputum collector 250.

Upon retraction of the catheter 220, sputum, phlegm and other undesired respiratory secretions are squeegeed from the external surface of the catheter 220 by the wiper seal 230. The diameter of the hole 233 is desirably sized slightly smaller than the outer diameter of the catheter 220 to facilitate this function. These undesired respiratory liquids are then directed into the sputum collector 250 where they are retained from leakage back toward the patient or out toward the respiratory practitioner. With the practitioner being equipped with protective hand ware and the patient being covered by a sterile field barrier under the retracted catheter 220, adequate protection from cross-contamination is thereby achieved.

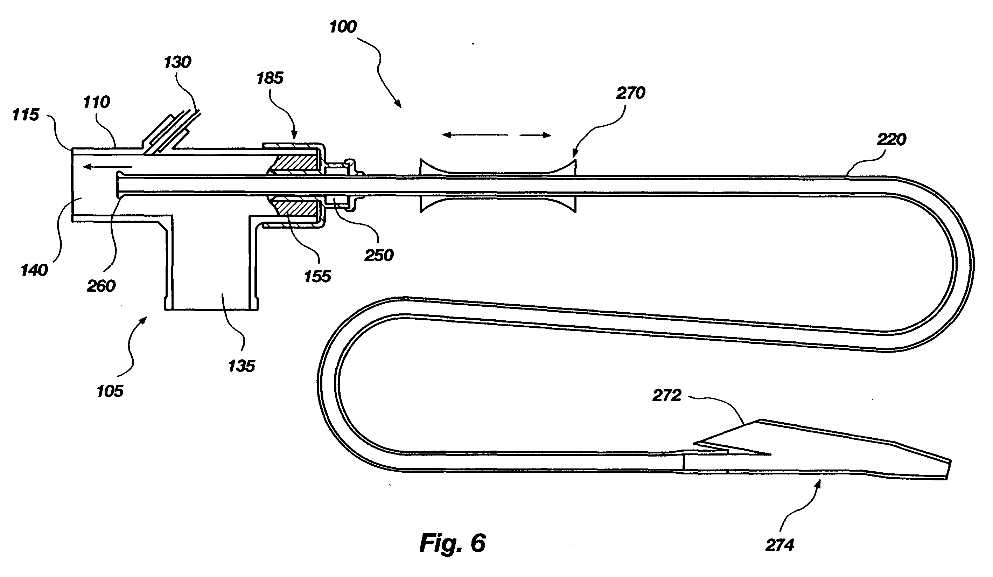

The catheter passageway 205 narrows at a reducing constrictor 255 near the catheter opening 215. A flare 260 at the catheter nozzle 265 catches against the reducing constrictor 255 upon retraction of the catheter 220. The embodiment illustrated by Figure 6 provides a catheter advancer 270. A practitioner can press the walls of the advancer 270 down upon a catheter 220 for selectively advancing and retracting the catheter 220. In this fashion, the practitioner can manipulate the catheter without touching it and without the need for special hand ware.

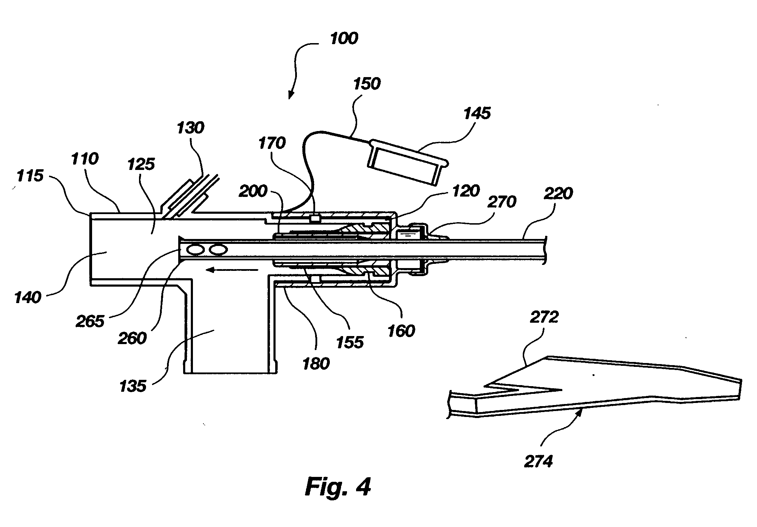

As best shown by Figures 4 and 6, the device 100 and its respective components allow a respiratory practitioner to maintain a closed-system within the single-patient, multiple use manifold assembly 105 throughout attachment of the catheter adapter 185 to the manifold assembly 105, evacuation of a medical patient through ambidextrous actuation of the vacuum pressure by selectively plugging an opening 272 in an actuator cylinder 274, retraction of the tracheal catheter 220 and detachment of the single-use, disposable catheter carrier 185. The practitioner may then, with minimal waste and cost, discard the catheter 220 and carrier structure 185 and place the cap 145 over the proximal end 120 of the manifold assembly 105 until next use, leaving no partially obstructed catheter, plastic envelope and complicated valve structure to interfere with other procedures and auxiliary equipment.

The present invention may be embodied in other specific forms. Further objects and advantages of this invention will become apparent from a consideration of the drawings and ensuing description of it. The described embodiments are to be considered in all respects only as illustrative, and not restrictive. The scope of the invention is, therefore, indicated by the appended claims, rather than by the foregoing description.

## Claims

1. A ventilation circuit adapter (100) for interfacing a suction catheter (220) with an intubation device, comprising: a manifold assembly (105), having:
a body (110) with an open interior (125), said body defining a ventilation port (135) in fluid communication with said open interior;
a distal end portion (115) of said body (110) being adapted to couple with said intubation device,
a proximal end portion (120) of said body being formed as an open port defined by a continuous wall, said proximal end portion being approximately axially aligned with said distal end portion (115) so that a catheter may be inserted through said proximal end portion (120) to exit from said distal end portion, and
a catheter carrier (185) for associating a catheter with said proximal end (120), said catheter carrier (185) being releasably connectable to said proximal end portion (120),
a directional barrier (155) carried by said proximal end portion (120), constructed and arranged to effect a seal against fluid flow through said open interior towards said proximal end portion but to pass a said catheter introduced through said proximal end portion (120), said adapter being **characterized by** said directional barrier (155) carrying detection means constructed and arranged to signal the presence of a tip of a catheter when said tip is moved to the proximity of said directional barrier from a location closer to said distal end portion.

2. An adapter according to Claim 1, wherein said directional barrier (155) is configured to be inserted in said proximal end portion (120) in sealing relationship with said open interior (125) and comprises a normally closed valving structure.

3. An adapter according to Claim 2, wherein said normally closed valve structure is constructed and arranged to effect a sliding seal with the external surface of a catheter as a said catheter is passed from said proximal end portion towards said distal end portion though said valve structure.

4. An adapter according to Claim 1, wherein said catheter carrier (185) comprises:
a leading end portion adapted to couple with said proximal end portion (120); and
a trailing end portion carrying a catheter introducer structure, said introducer structure being constructed and arranged to interface with said directional barrier when said leading end portion is coupled with said proximal end portion.

5. An adapter according to Claim 4, wherein said introducer structure comprises:
an introducer tip (200) constructed as a continuous wall to define:
an interior passageway configured to receive and pass a catheter; and
an exterior surface configured to interface with said directional barrier.

6. An adapter according to Claim 5, further comprising: a sputum fluid collector carried by said trailing end portion and including:
a chamber positioned in approximate axial alignment with said distal end portion opposite said manifold with respect to said leading end portion;
a proximal closing member for said chamber with an entry approximately axially aligned with said introducer tip, said closing member being structured to effect a sliding seal with the exterior surface of a catheter.

7. An adapter according to Claim 6, wherein said chamber is structured as a hollow cylindrical extension from said trailing end portion, and said closing member is structured as an end panel of said extension with a central opening adapted to receive a catheter in friction-slipping engagement.

8. An adapter according to Claim 1, where:
the catheter carrier, includes:
a leading end portion adapted to couple with said proximal end portion, and
a trailing end portion carrying a catheter introducer structure; and
the directional barrier is constructed and arranged
to effect a seal between said proximal end portion and said carrier,
said introducer structure being constructed and arranged to interface with said directional barrier when said leading end portion is coupled with said proximal end portion.

9. An adapter according to Claim 8, wherein said introducer structure comprises:
an introducer tip constructed as a continuous wall to define:
an interior passageway configured to receive and pass a catheter; and
an exterior surface configured to interface with said directional barrier.

10. An adapter according to Claim 1, further including a catheter (220) positioned through said catheter carrier (185), said open interior and said distal end portion.

11. An adapter according to Claim 10, wherein said catheter carries indicia, and said manifold assembly body includes a transparent window portion positioned so that said indicia are viewable through said window portion as said catheter is moved parallel the axis of said manifold assembly body.

12. An adapter according to Claim 11, wherein said indicia comprise markings carried by the exterior surface of said catheter.

13. An adapter according to Claim 12, wherein said manifold assembly includes a ventilation port (135) in fluid flow communication with said open interior, said ventilation port having a central axis transverse the central axes of said proximal end portion and said distal end portion.

14. An adapter according to Claim 1, wherein said manifold assembly includes a lavage port in fluid flow communication with said open interior, said lavage port having a central axis transverse said central axes of said proximal end portion and said distal end portion.

15. An adapter according to Claim 1, further comprising a positive stop mechanism (255) arranged to prevent retraction of said catheter from catheter carrier (185)

## Patentansprüche

1. Ein Beatmungskreislaufadapter (100) zum Anschließen eines Saugkatheters (220) an eine Intubationsvorrichtung, umfassend:
einen Verteileraufbau (105) mit:
einem einen offenen Innenraum (125) aufweisenden Körper (110), welcher einen Ventilationsanschluss (135) bildet, der in Fluidverbindung mit dem offenen Innenraum ist;
einem distalen Endabschnitt (115) des Körpers (110), welcher zur Kopplung mit der Intubationsvorrichtung ausgebildet ist,
einem proximalen Endabschnitt (120) des Körpers, welcher als ein durch eine kontinuierliche Wand umschriebener offener Anschluss ausgebildet ist und etwa axial zu dem distalen Endabschnitt (115) ausgerichtet ist, so dass ein Katheter durch den proximalen Endabschnitt (120) eingeführt werden kann, um beim distalen Endabschnitt auszutreten, und
einen Katheterträger (185), welcher lösbar mit dem proximalen Endabschnitt verbindbar ist, zum Zuordnen eines Katheters zu dem proximalen Ende (120),
eine von dem proximalen Endabschnitt (120) getragene Richtungsschranke (155), welche in der Weise konstruiert und angeordnet ist, dass sie eine Abdichtung gegen Flüssigkeitsfluss durch den offenen Innenraum in Richtung des proximalen Endabschnitts bewirkt, aber einen Katheter, welcher durch den proximalen Endabschnitt (120) eingeführt wird, passieren lässt,
**dadurch gekennzeichnet, dass** die Richtungsschranke (155) Detektionsmittel aufweist, welche so ausgebildet und angeordnet sind, dass sie das Vorhandensein einer Spitze eines Katheters anzeigen, wenn die Spitze von einem näher zu dem distalen Endabschnitt liegenden Ort in die Nähe der Richtungsschranke bewegt wird.

2. Adapter nach Anspruch 1,
bei dem die Richtungsschranke (155) so ausgebildet ist, dass sie in den proximalen Endabschnitt (120) abdichtend gegen den offenen Innenraum (125) eingesetzt werden kann und eine grundsätzlich geschlossene Ventilstruktur umfasst.

3. Adapter nach Anspruch 2,
bei dem die grundsätzlich geschlossene Ventilstruktur in der Weise ausgebildet und angeordnet ist, dass sie eine Gleitabdichtung mit der äußeren Oberfläche eines Katheters bewirkt, wenn der Katheter sich von dem proximalen Endabschnitt in Richtung des distalen Endabschnitts durch die Ventilstruktur bewegt.

4. Adapter nach Anspruch 1,
bei dem der Katheterträger (185) umfasst:
Einen Führungsendabschnitt, welcher zur Kopplung mit dem proximalen Endabschnitt (120) ausgebildet ist, und
einen hinteren Endabschnitt, welcher eine Kathetereinführungsstruktur trägt, welche in der Weise ausgebildet und angeordnet ist, dass sie an die Richtungsschranke angeschiossen wird, wenn der Führungsendabschnitt mit dem proximalen Endabschnitt gekoppelt ist.

5. Adapter nach Anspruch 4,
bei dem die Einführungsstruktur umfasst:
Eine Einführungsspitze (200), welche als kontinuierliche Wand ausgebildet ist und eine zur Aufnahme und Durchführung eines Katheters ausgebildete innere Durchführung und eine zum Anschluss an die Richtungsschranke ausgebildete äußere Oberfläche definiert.

6. Adapter nach Anspruch 5,
weiterhin umfassend:
Einen Sputumsammler, welcher durch den hinteren Endabschnitt getragen wird und beinhaltet:
Eine Kammer, welche in etwa axialer Ausrichtung zu dem distalen Endabschnitt gegenüber dem Verteiler in Bezug auf den Führungsendabschnitt angeordnet ist;
ein proximales Verschlusselement für die Kammer mit einem Eingang, welcher etwa axial zu der Einführspitze ausgerichtet ist und so ausgebildet ist, dass es eine Gleitabdichtung mit der äußeren Oberfläche eines Katheters bewirkt.

7. Adapter nach Anspruch 6,
bei dem die Kammer als hohle, zylindrische Verlängerung des hinteren Endabschnitts ausgebildet ist und das Schließelement als Endwand der Verlängerung ausgebildet ist mit einer zentralen Öffnung, welche zur Aufnahme eines Katheters in reibend-gleitender Weise ausgebildet ist.

8. Adapter nach Anspruch 1,
bei dem der Katheterträger umfasst:
Einen Führungsendabschnitt, welcher zur Kopplung mit dem proximalen Endabschnitt ausgebildet ist und
einen hinteren Endabschnitt, welcher eine Kathetereinführungsstruktur trägt; und
bei dem die Richtungsschranke in der Weise ausgebildet und angeordnet ist, dass sie eine Dichtung zwischen dem proximalen Endabschnitt und dem Träger bewirkt, und die Einführungsstruktur in der Weise ausgebildet und angeordnet ist, dass sie an die Richtungsschranke angeschlossen wird, wenn der Führungsendabschnitt mit dem proximalen Endabschnitt gekoppelt wird.

9. Adapter nach Anspruch 8,
bei dem die Einführungsstruktur umfasst:
Eine Ausführungsspitze (200), welche aus kontinuierliche Wand ausgebildet ist und eine zur Aufnahme und Durchführung eines Katheters ausgebildete innere Durchführung und eine zum Anschiuss an die Richtungsschranke ausgebildete äußere Oberfläche definiert.

10. Adapter nach Anspruch 1,
weiterhin umfassend einen Katheter (220), welcher durch den Katheterträger (185), den offenen Innenraum und den distalen Endabschnitt positioniert ist.

11. Adapter nach Anspruch 10,
bei dem der Katheter Indikatoren trägt und der Verteileraufbaukörper einen transparenten Fensterabschnitt beinhaltet, welcher so positioniert ist, dass die Indikatoren durch den Fensterabschnitt sichtbar sind, wenn der Katheter parallel zu der Achse des Verteileraufbaukörpers bewegt wird.

12. Adapter nach Anspruch 11,
bei dem die Indikatoren Markierungen umfassen, welche auf der äußeren Oberfläche des Katheters befestigt sind.

13. Adapter nach Anspruch 12,
bei dem der Verteileraufbau einen Ventilationsanschluss umfasst, welcher in Fluidverbindung mit dem offenen Innenraum ist und dessen Mittelachse quer zur Mittelachse des proximalen Endabschnitts und des distalen Endabschnitts liegt.

14. Adapter nach Anspruch 1,
bei dem der Verteileraufbau einen Spülanschluss beinhaltet, welcher in Fluidverbindung mit dem offenen Innenraum ist und dessen Mittelachse quer zur Mittelachse des proximalen Endabschnitts und des distalen Endabschnitts liegt.

15. Adapter nach Anspruch 1,
weiterhin umfassend einen positiven Stoppmechanismus (255), welcher geeignet ist, den Rückzug des Katheters aus dem Katheterträger (185) zu verhindern.

## Revendications

1. Embout de circuit de ventilation (100) pour servir d'interface entre un cathéter d'aspiration (220) et un dispositif d'intubation, comprenant : un ensemble distributeur (105), ayant :
un corps (110) présentant un intérieur ouvert (125), ledit corps définissant un port de ventilation (135) dans la communication des liquides avec ledit intérieur ouvert ;
une partie d'extrémité distale (115) dudit corps (110) étant adaptée à être couplée audit dispositif d'intubation ;
une partie d'extrémité proximale (120) dudit corps étant formée comme un port ouvert défini par une paroi continue, ladite partie d'extrémité proximale étant approximativement alignée sur l'axe de ladite partie d'extrémité distale (115) de sorte qu'un cathéter puisse être inséré à travers ladite partie d'extrémité proximale (120) pour ressortir depuis ladite partie d'extrémité distale ; et
un tube de cathéter (185) pour associer un cathéter à ladite partie d'extrémité proximale (120), ledit tube de cathéter (185) étant relié de manière amovible à ladite partie d'extrémité proximale (120) ;
une couche étanche de direction (155) portée par ladite partie d'extrémité proximale (120), conçue et positionnée pour déposer un joint d'étanchéité contre le courant de liquides s'écoulant à travers ledit intérieur ouvert en direction de ladite partie d'extrémité proximale, mais, pour laisser passer ledit cathéter introduit à travers ladite partie d'extrémité proximale (120), ledit embout étant **caractérisé par** ladite couche étanche de direction (155) portant des moyens de détection conçus et positionnés pour signaler la présence d'un intubateur de cathéter lorsque ledit intubateur est déplacé à proximité de ladite couche étanche de direction depuis un point plus proche de ladite partie d'extrémité distale.

2. Embout selon la revendication 1, dans lequel ladite couche étanche de direction (155) est configurée pour être insérée dans ladite partie d'extrémité proximaie (120) dans une relation d'étanchéité avec ledit intérieur ouvert (125) et comprend une structure de soupape normalement fermée.

3. Embout selon la revendication 2, dans lequel ladite structure de soupape normalement fermée est conçue et positionnée pour déposer par glissement un joint sur la surface externe d'un cathéter lorsqu'un dit cathéter est introduit depuis ladite partie d'extrémité proximale en direction de ladite partie d'extrémité distale à travers ladite structure de soupape.

4. Embout selon la revendication 1, dans lequel ledit tube de cathéter (185) comprend :
une partie d'extrémité d'introduction adaptée à être couplée à ladite partie d'extrémité proximale (120) ; et
une partie d'extrémité postérieure portant une structure d'intubateur de cathéter, ladite structure d'intubateur de cathéter étant conçue et positionnée pour servir d'interface avec ladite couche étanche de direction lorsqu'une partie d'extrémité d'introduction est couplée à ladite partie d'extrémité proximale.

5. Embout selon la revendication 4, dans lequel ladite structure d'intubateur comprend :
un intubateur (200) conçu comme une paroi continue de manière à définir :
une voie de passage intérieure configurée pour recevoir et laisser passer un cathéter ; et une surface extérieure configurée pour servir d'interface avec ladite couche étanche de direction.

6. Embout selon la revendication 5, comprenant en outre :
un collecteur de sécrétions liquides expectorées porté par ladite partie d'extrémité postérieure et comprenant :
un compartiment positionné suivant un alignement axial approximatif de ladite partie d'extrémité distale opposée audit distributeur par rapport à ladite partie d'extrémité d'introduction ;
un membre de fermeture proximal pour ledit compartiment avec une entrée approximativement alignée sur l'axe dudit intubateur, ledit membre de fermeture étant structuré pour déposer par glissement un joint sur la surface extérieure d'un cathéter.

7. Embout selon la revendication 6, dans lequel ledit compartiment est structuré comme un cylindre d'extension creux depuis ladite partie d'extrémité postérieure, et ledit membre de fermeture est structuré comme un panneau terminal de ladite extension présentant une ouverture centrale adaptée pour recevoir un cathéter venant en prise par frottement - glissement.

8. Embout selon la revendication 1, dans lequel le cathéter comprend :
une partie d'extrémité d'introduction adaptée pour être couplée à ladite partie d'extrémité proximale, et
une partie d'extrémité postérieure portant une structure d'intubateur de cathéter ; et la couche étanche de direction est conçue et positionnée pour déposer un joint entre ladite partie d'extrémité proximale et ledit tube ; ladite structure d'intubateur étant conçue et disposée pour servir d'interface avec ladite couche étanche de direction lorsque ladite partie d'extrémité d'introduction est couplée à ladite partie d'extrémité proximale.

9. Embout selon la revendication 8, dans lequel ladite structure d'intubateur comprend :
un intubateur conçu comme une paroi continue de manière à définir :
une voie de passage intérieure configurée pour recevoir et laisser passer un cathéter ; et une surface extérieure configurée pour servir d'interface avec ladite couche étanche de direction.

10. Embout selon la revendication 1, comprenant en outre un cathéter (220) positionné à travers ledit tube de cathéter (185), ledit intérieur ouvert et ladite partie d'extrémité distale.

11. Embout selon la revendication 10, dans lequel ledit cathéter porte des indices, et ledit corps de l'ensemble distributeur comprend une partie fenêtre transparente positionnée de sorte que les indices soient visibles à travers ladite partie fenêtre lorsque ledit cathéter est déplacé parallèlement à l'axe dudit corps de l'ensemble distributeur.

12. Embout selon la revendication 11, dans lequel lesdits indices comprennent des marquages inscrits sur la surface extérieure dudit cathéter.

13. Embout selon la revendication 12, dans lequel ledit ensemble distributeur comprend un port de ventilation (135) dans la communication du courant de liquides avec ledit intérieur ouvert, ledit port de ventilation ayant un axe central transversal à l'axe central de ladite partie d'extrémité proximale et de ladite partie d'extrémité distale.

14. Embout selon la revendication 1, dans lequel ledit ensemble distributeur comprend un port de lavage dans la communication du courant de liquides avec ledit intérieur ouvert, ledit port de lavage ayant un axe central transversal à l'axe central de ladite partie d'extrémité proximale et de ladite partie d'extrémité distale.

15. Embout selon la revendication 1, comprenant en outre un mécanisme de butée fixe (255) positionné de manière à empêcher la rétraction dudit cathéter du tube de cathéter (185).
